# EUROPÄISCHE PATENTSCHRIFT

(11) **EP 1 231 875 B1**
(45) Veröffentlichungstag und Bekanntmachung des Hinweises auf die Patenterteilung: **15.09.2004**
(21) Anmeldenummer: 99967919.4
(22) Anmeldetag: 22.12.1999
(51) Int. Cl.: A61F 7/00

(54) **EINRICHTUNG ZUR LOKALEN THERMISCHEN BEHANDLUNG VON INSEKTENSTICHEN UND -BISSEN**
DEVICE FOR LOCAL THERMAL TREATMENT OF INSECT STINGS AND BITES
DISPOSITIF DE TRAITEMENT THERMIQUE LOCAL DE PIQURES ET DE MORSURES D'INSECTES

(30) Priorität: 11.11.1999 DE 19954424
(43) Veröffentlichungstag der Anmeldung: 21.08.2002
(73) Patentinhaber: BSM-Bionic Solution Management GmbH, 17489 Greifswald (DE)
(72) Erfinder: Kruppa, Rainer, 17493 Greifswald (DE); Enderlein, Dietmar, Prof. Dr.med., 17489 Greifswald (DE); Schuldzig, Hansgeorg, 21635 York (DE)
(74) Vertreter: Kietzmann, Manfred
(86) Internationale Anmeldenummer: PCT/DE1999/004115
(87) Internationale Veröffentlichungsnummer: WO 2001/034074

(56) Entgegenhaltungen:
- EP-A- 0 312 413
- EP-A- 0 607 472
- DE-A- 19 807 453
- FR-A- 2 746 296

## Beschreibung

Die Erfindung betrifft ein Handgerät, das zur Behandlung der Folgen von Insektenstichen und -bissen verwendet wird.

### [Stand der Technik]

Stiche oder Bisse von einem Insekt wie einer Stechmücke, Bremse, Laus, einem Floh, einer Wanze oder Ameise, sind im allgemeinen harmlos. Ein Insektenstich führt zu juckenden, auch schmerzenden Anschwellungen, die oft bald wieder abklingen. An der Stichstelle kommt es zu Rötung, Quaddelbildung und schmerzhafter Schwellung. Solche Stiche können aber gefährlich werden, wenn das Insekt Krankheitserreger überträgt. Darüber hinaus können die Folgen eines Insektenstichs für den Betroffenen mit langwierigen und lästigen Erscheinungen, wie etwa offenen und aufgekratzten Stellen an der Einstichstelle, verbunden sein.

Stiche von Bienen, Wespen, Hornissen oder Hummeln schaden durch Gift, dessen Wirkung besonders von der Empfindlichkeit des Gestochenen und vom Ort des Einstichs abhängt.

Zu den bekannten Methoden der Vorbeugung gegen Insektenstiche gehört:
- mechanische Mückenabwehrmittel (Gazefenster; Moskitonetze; dichtgewebte Stoffe als Bekleidung, in die der Stechrüssel nicht eindringen kann),
- Geräte, die die Laute paarungswilliger Männchen nachahmen, worauf die begatteten blutsaugenden Weibchen flüchten,
- insektenvertreibende Mittel (stark riechende, sich schnell auf der Haut verflüchtigende Öle wie z. B. Nelkenöl und Senföl).

Alle bekannten Mittel und Methoden der Vorbeugung können letztlich Insektenstiche und Bisse nicht verhindern.

Ist es zum Stich oder Biß gekommen, so wird der Juckreiz oft durch antiallergene Salben oder Alkohol gemildert. Bei Bienen- oder Wespenstichen wird der Einstichkanal nach dem Entfernen des zurückgebliebenen Stachels mit Salmiakgeist betupft. Die Behandlung der Einstichstelle und der Quaddeln führt in der Regel nur zu einer vorübergehenden Linderung der Symptome. Ein sicheres und dauerhaftes Abklingen der Schwellungen und des Juckreizes ist damit nicht zu erreichen.

Es ist auch schon eine Vorrichtung bekannt, die mit intensiver Wärmestrahlung auf die Einstichstelle einwirkt. Die Wärme wird mit Hilfe einer Glühbirne erzeugt, die durch einen Startknopf betätigt wird. Der Startknopf wird dabei solange gedrückt gehalten, bis die maximale Behandlungstemperatur von etwa 70° C erreicht ist. Danach soll die Behandlung beendet werden.

Die Wärmestrahlung soll die in die Hautschichten gelangten thermolabilen Gifte neutralisieren und beseitigen und somit Juckreiz und Schwellungen verhindern.

Von Nachteil ist, daß mit dieser Vorrichtung der Wärmeeintrag weder in Bezug auf die Höhe der Temperatur noch in Bezug auf die Dauer der Einwirkung exakt dosiert werden kann, sondern vom Geschick und den Erfahrungen des Anwenders abhängt.

Dadurch kann es leicht entweder zum Ausbleiben des Erfolges oder zum Überschreiten der schadlos erträglichen Temperatur kommen und damit zu zusätzlichen Hautreizungen kommen.

FR-A-2 746 296 offenbart eine Einrichtung zur thermischen Behandlung von Insektenstichen und-bissen mit den Merkmalen des Oberbegriffs des Anspruchs 1.

### [Aufgabe der Erfindung]

Der Erfindung liegt daher die Aufgabe zugrunde, eine Vorrichtung zur thermischen Behandlung von Insektenstichen und - bissen zu schaffen, mit der unabhängig von den äußeren Bedingungen die für das Neutralisieren der thermolabilen Insektengifte erforderliche Wärmemenge eingebracht wird, ohne daß es dabei zu auf die Wärmeeinwirkung zurückzuführenden Hautreizungen kommt.

Gelöst wird diese Aufgabe durch eine Einrichtung mit den Merkmalen des Anspruchs 1. Vorteilhafte Ausgestaltungen der Erfindung sind in den Unteransprüchen angegeben.

### [Beispiele]

Im folgenden wird die Erfindung anhand eines Ausführungsbeispiels erläutert.

Es zeigen:
- Figur 1: Blockschaltbild der erfindungsgemäßen Einrichtung,
- Figur 2: ein Temperatur - Zeit - Diagramm der Temperatur an der Heizplatte für vier verschiedene Maximaltemperaturen,
- Figur 3: ein Blockschaltbild eines Heizchips.

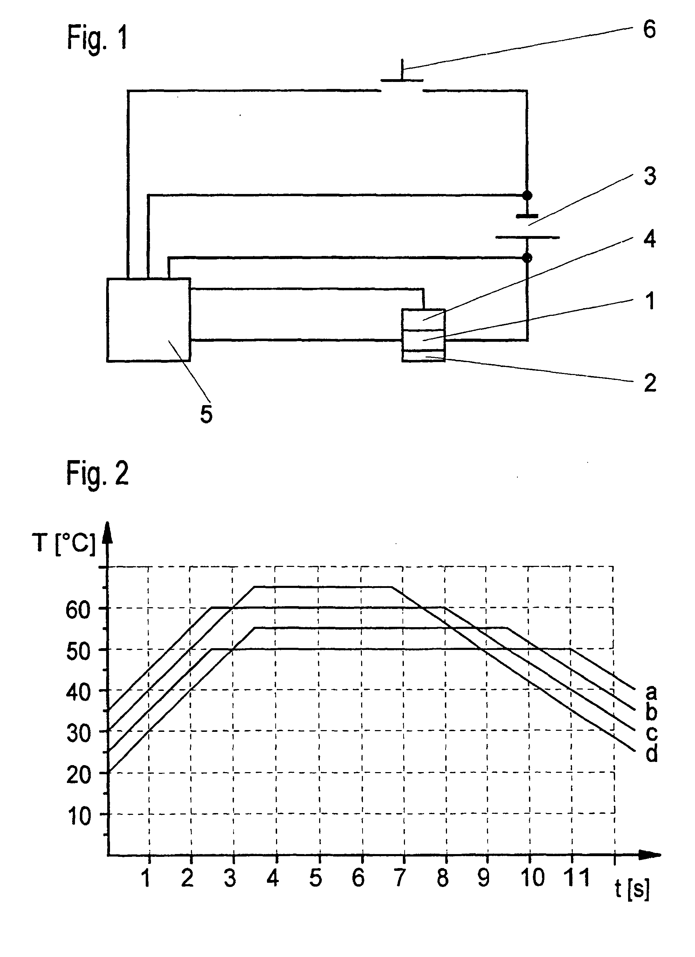

Die Einrichtung gemäß Figur 1 besteht aus einem Heizelement 1, das als elektrische Heizplatte 2 in Form einer runden Scheibe mit einem Durchmesser von etwa 5 mm ausgeführt ist. Das Heizelement 1 wird durch eine Spannungsquelle 3 gespeist. Mit dem Heizelement 1 verbunden ist ein Temperatursensor 4. Das vom Temperatursensor 4 erzeugte elektrische Signal wird einer Steuereinrichtung 5 zugeführt, mit der die Temperatur und die Dauer der Aufrechterhaltung der maximalen Temperatur der Heizplatte 2 gesteuert wird.

Durch das Betätigen eines Tasters 6 wird mittels der Steuereinrichtung 5 im Heizelement 1 die Aufheizphase der Heizplatte 2 gestartet. Sobald das vom Sensor 4 abgegebene elektrische Signal die der vorgegebenen maximalen Temperatur an der Heizplatte 2 entsprechende Größe erreicht hat, beginnt die Heizphase. Dazu wird in der Steuereinrichtung 5, die eine Zeit- und eine Temperaturregelung enthält, die Zeitsteuerung ausgelöst. Während der vorgegebenen Dauer der Heizphase, die der Behandlungsdauer entspricht, gewährleistet die Temperaturregelung die Aufrechterhaltung der maximalen Temperatur und verhindert gleichzeitig deren Überschreiten. Nach dem Ablauf der Heizphase wird der Heizvorgang beendet und Temperatur der Heizplatte 2 paßt sich der Umgebungstemperatur an.

Die Heizplatte 2 wird in der Aufheizphase auf eine maximale Temperatur aus einem Bereich von 50 bis 65°C, vorzugsweise 55 bis 60°C, erhitzt, wobei eine Toleranz von ± 3 °C eingehalten wird. In der auf die Aufheizphase folgenden Heizphase, die der Behandlungsdauer entspricht, wird die maximale Temperatur für einen Zeitraum von 2 bis 12 s, vorzugsweise 3 bis 6 s, aufrechterhalten. Dabei ist die Dauer der Heizphase umgekehrt proportional der Höhe der maximalen Temperatur. Mit dem Ende der Heizphase wird der Heizvorgang abgebrochen und es beginnt die Abkühlphase. Das Ende des Heizphase und somit der Behandlung kann durch ein Signal angezeigt werden. Die Einrichtung ist nunmehr für weitere Behandlungen bereit.

Figur 2 zeigt den Temperaturverlauf an der Heizplatte 2 in einem Temperatur - Zeit - Diagramm für vier verschiedene maximale Temperaturen.

Bei dem in Kurve a dargestellten Verlauf beginnt die Aufheizphase bei einer Ausgangstemperatur von 25 °C und dauert bis zum Erreichen der vorgegebenen Maximaltemperatur von 50 °C 2,5 s. Mit dem Erreichen der Maximaltemperatur beginnt die Heizphase, die hier 8,5 s dauert, und innerhalb derer die Maximaltemperatur innerhalb des Toleranzbereichs aufrechterhalten wird. Während der Dauer der Heizphase wird Wärme in den von der Heizplatte berührten Einstich der Haut eingetragen und es erfolgt die Neutralisierung der im Einstich befindlichen thermolabilen Insektengifte. Mit dem Ablauf der Heizphase endet das Heizen und die Temperatur der Heizplatte 2 paßt sich wieder der Umgebungstemperatur an.

Bei dem in Kurve b dargestellten Verlauf beginnt die Aufheizphase bei einer Ausgangstemperatur von 20 °C und dauert bis zum Erreichen der vorgegebenen Maximaltemperatur von 55 °C 3,5 s. Die Heizphase dauert hier 6 s.

Bei dem in Kurve c dargestellten Verlauf beginnt die Aufheizphase bei einer Ausgangstemperatur von 35 °C und dauert bis zum Erreichen der vorgegebenen Maximaltemperatur von 60 °C 2,5 s. Die Heizphase dauert hier 5,5 s.

Bei dem in Kurve d dargestellten Verlauf beginnt die Aufheizphase bei einer Ausgangstemperatur von 30 °C und dauert bis zum Erreichen der vorgegebenen Maximaltemperatur von 65 °C 3,5 s. Die Heizphase dauert hier 3,25 s.

Das Einbringen der Wärmemenge in den Einstich erfolgt unabhängig von der Umgebungstemperatur und der Oberflächentemperatur der Haut, da die Heizphase erst mit dem Erreichen der maximalen Temperatur beginnt und die Temperatur der Heizplatte 2 über die gesamte Dauer der Heizphase durch die Temperaturregelung ständig aufrechterhalten wird. Auf diese Weise ist ein exakter Eintrag der für das Neutralisieren der thermolabilen Gifte erforderlichen Wärmemenge in einem Temperaturbereich möglich, bei dem Schädigungen der Haut durch diesen Wärmeeintrag nicht zu befürchten sind.

Tests haben ergeben, daß, wenn die Behandlung unmittelbar nach dem Stich erfolgt, bei einer Maximaltemperatur von 55 °C Mückenstiche erfolgreich entaktiviert werden können. Die Einwirkdauer ist bei einer Temperatur von 55 °C unkritisch, so daß die Behandlung bei dieser Temperatur im Bedarfsfall mehrfach wiederholt werden kann. Darüber hinaus wird infolge der betäubenden Wirkung von Insektengift eine Wärmebehandlung der Haut an der Einstichstelle als deutlich weniger unangenehm empfunden als in anderen Bereichen.

Bei einer Maximaltemperatur von etwa 60 °C sollte die Heizphase 5 s nicht überschreiten, da es sonst auf empfindlichen Hautpartien zu Hautreaktionen infolge des Wärmeeintrages kommt.

Beträgt die Maximaltemperatur 65 °C, sollte die Heizphase 3,5 s nicht überschreiten, da es sonst zu Hautreaktionen infolge des Wärmeeintrages kommt.

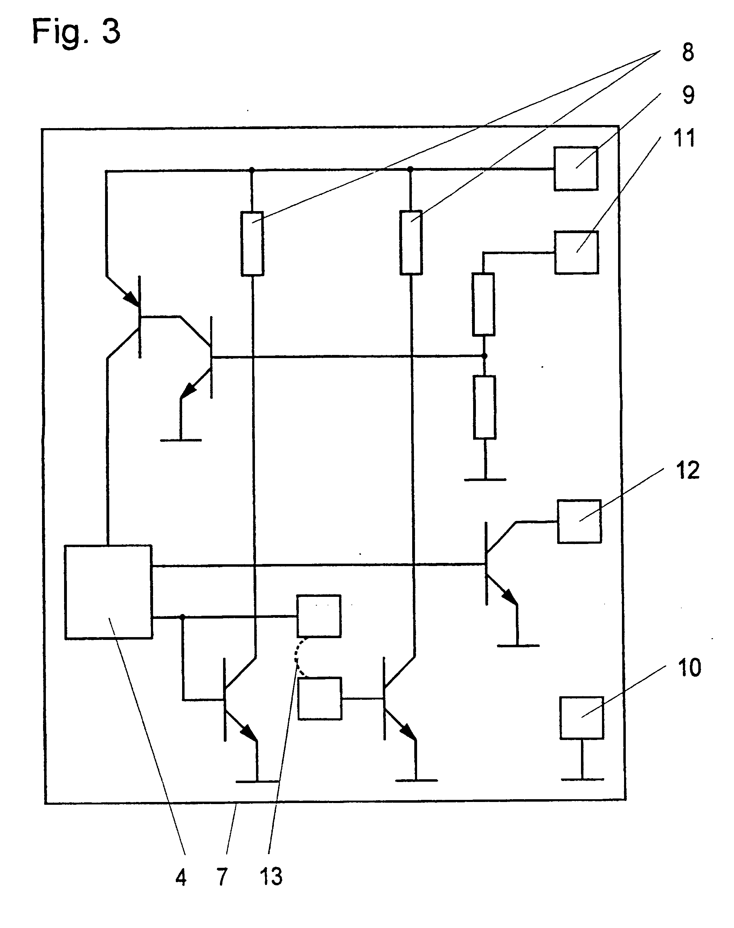

Das Heizelement 1 mit Heizplatte 2 kann als integrierte Schaltung eines Siliziumchips ausgeführt sein. Ein Blockschaltbild des Heizchip wird in Figur 3 gezeigt. Der Heizchip 7 ist mit einem Temperatursensor 4, einen Schalttransistor und einem Heizer 8 in Form einer Widerstandsmatrix ausgestattet. Die Batteriespannung wird am Anschluß 9 in den Chip eingeleitet und am Anschluß 10 ist der Chip mit der Masse verbunden. Am Anschluß 11 wird das Steuersignal eingeleitet und am Anschluß 12 liegt ein Signal an, das dem aktuellen Regelzustand (Status) entspricht.

Bei Anlegen eines Steuersignals (Eingangsspannung high ≥ 2 V) am Anschluß 11 wird der Schaltkreis aktiviert und die Heizung eingeschaltet. Dazu wird über den Schalttransistor ein Strom über den großflächigen integrierten Widerstand 8 geleitet. Bei Erreichen der vorgegebenen Maximaltemperatur setzt die Regelung ein. Die Regelspannung wird versteilert und an ein Ausgangspin 12 geführt (beim Erreichen der Maximaltemperatur wird der Ausgang "low"). Die Heizleistung beträgt bei 9 V ca. 0.8 W. Bei Bedarf kann die Heizleistung durch eine zusätzliche Bonddrahtbrücke 13 verdoppelt werden.

### [Bezugszeichenliste]

- 1: Heizelement
- 2: Heizplatte
- 3: Spannungsquelle
- 4: Temperatursensor
- 5: Steuereinheit
- 6: Taster
- 7: Heizchip
- 8: Heizer
- 9: Anschluß für Batteriespannung
- 10: Masseanschluß
- 11: Anschluß für Steuersignal
- 12: Status-Anschluß
- 13: Bonddrahtbrücke

## Patentansprüche

1. Einrichtung zur lokalen thermischen Behandlung von Insektenstichen und -bissen, die mittels Wärmeeintrag auf die Einstichstelle einwirkt, wobei
a) die Einrichtung ein Heizelement (1) umfaßt, das als elektrische Heizplatte (2) ausgeführt ist, die durch eine Spannungsquelle (3) gespeist wird,
b) die Heizplatte (2) in einer Aufheizphase auf eine maximale Temperatur aus einem Bereich von 50 bis 65°C, vorzugsweise 55 bis 60°C, erhitzbar ist und die maximale Temperatur in einer Heizphase für einen Zeitraum von 2 bis 12 s, vorzugsweise 3 bis 6 s, aufrechterhalten werden kann,
c) mit dem Heizelement (1) ein Temperatursensor (4) und eine Steuereinrichtung verbunden sind, wobei das vom Temperatursensor (4) erzeugte elektrische Signal der Steuereinrichtung (5) zugeführt wird, **dadurch gekennzeichnet, daß** mit der Steuereinrichtung das Aufheizen der Heizplatte (2) auf die maximale Temperatur und die Dauer der Aufrechterhaltung der maximalen Temperatur gesteuert wird.

2. Einrichtung nach Anspruch 1, **dadurch gekennzeichnet, daß** während der Heizphase die Maximaltemperatur von 50 °C der Heizplatte (2) für eine Dauer von 8,5 s aufrechterhalten wird.

3. Einrichtung nach Anspruch 1, **dadurch gekennzeichnet, daß** während der Heizphase die Maximaltemperatur von 55 °C der Heizplatte (2) für eine Dauer von 6 s aufrechterhalten wird.

4. Einrichtung nach Anspruch 1, **dadurch gekennzeichnet, daß** während der Heizphase die Maximaltemperatur von 60 °C der Heizplatte (2) für eine Dauer 5,5 s aufrechterhalten wird.

5. Einrichtung nach Anspruch 1, **dadurch gekennzeichnet, daß** während der Heizphase die Maximaltemperatur von 65 °C der Heizplatte (2) für eine Dauer von 3,25 s aufrechterhalten wird.

6. Einrichtung nach den Ansprüchen 1 bis 5, **dadurch gekennzeichnet, daß** die Maximaltemperatur während der Heizphase mit einer Toleranz von ± 3 °C eingehalten wird.

7. Einrichtung nach den Ansprüchen 1 bis 5, **dadurch gekennzeichnet, daß** das Heizelement (1) und der Temperatursensor (4) in einer integrierten Schaltung eines Siliziumchips (7) mit Schalttransistor enthalten sind, wobei das Heizelement (1) als Widerstandsmatrix (8) ausgeführt ist.

## Claims

1. Device for the local thermal treatment of insect stings and insect bites that acts on the puncture point by means of the introduction of heat,
a) the device comprising a heating element (1) that is designed as an electrical hotplate (2) supplied by a voltage source (3),
b) the hotplate(2) being heatable in a heating-up phase to a maximum temperature in a range from 50 to 65°C, preferably 55 to 60°C, and the maximum temperature being maintainable in a heating phase for a time interval ranging from 2 to 12 s, preferably 3 to 6 s,
c) a temperature sensor (4) and a control device being connected to the heating element (1), the electrical signal generated by the temperature sensor (4) being routed to the control device (5), **characterized in that** the control device controls the heating-up of the hotplate (2) to the maximum temperature and the duration of the maintenance of the maximum temperature.

2. Device according to Claim 1, **characterized in that**, during the heating phase, the maximum temperature of 50°C of the hotplate (2) is maintained for a duration of 8.5 s.

3. Device according to Claim 1, **characterized in that**, during the heating phase, the maximum temperature of 55°C of the hotplate (2) is maintained for a duration of 6 s.

4. Device according to Claim 1, **characterized in that**, during the heating phase, the maximum temperature of 60°C of the hotplate (2) is maintained for a duration of 5.5 s.

5. Device according to Claim 1, **characterized in that**, during the heating phase the maximum temperature of 65°C of the hotplate (2) is maintained for a duration of 3.25 s.

6. Device according to Claims 1 to 5, **characterized in that** the maximum temperature is maintained during the heating phase with a tolerance of ± 3°C.

7. Device according to Claims 1 to 5, **characterized in that** the heating element (1) and the temperature sensor (4) are contained in an integrated circuit of a silicon chip (7) comprising a switching transistor, the heating element (1) being designed as a resistor matrix (8).

## Revendications

1. Appareil de traitement thermique local de piqûres et de morsures d'insectes qui agit par apport de chaleur à l'endroit touché,
a) cet appareil comprenant un élément chauffant (1) qui est constitué d'une plaque chauffante électrique (2) qui est alimentée par une source de tension (3),
b) la plaque chauffante (2) pouvant, dans une phase de chauffage, être chauffée à une température maximale comprise entre 50 et 65 °C, de préférence entre 55 et 60 °C, et cette température maximale pouvant, dans une phase de chauffage, être maintenue pendant une durée de 2 à 12 s, de préférence de 3 à 6 s,
c) à l'élément chauffant (1) étant reliés un capteur de température (4) et un dispositif de commande (5), le signal électrique produit par le capteur de température (4) étant envoyé au dispositif de commande (5),
**caractérisé par le fait que** le dispositif de commande commande le chauffage de la plaque chauffante (2) à la température maximale et la durée de maintien de cette température.

2. Appareil selon la revendication 1, **caractérisé par le fait que** pendant la phase de chauffage, la température maximale de 50 °C de la plaque chauffante (2) est maintenue pendant une durée de 8,5 s.

3. Appareil selon la revendication 1, **caractérisé par le fait que** pendant la phase de chauffage, la température maximale de 55 °C de la plaque chauffante (2) est maintenue pendant une durée de 6 s.

4. Appareil selon la revendication 1, **caractérisé par le fait que** pendant la phase de chauffage, la température maximale de 60 °C de la plaque chauffante (2) est maintenue pendant une durée de 5,5 s.

5. Appareil selon la revendication 1, **caractérisé par le fait que** pendant la phase de chauffage, la température maximale de 65 °C de la plaque chauffante (2) est maintenue pendant une durée de 3,25 s.

6. Appareil selon les revendications 1 à 5, **caractérisé par le fait que** la température maximale est maintenue pendant la phase de chauffage avec une tolérance de ± 3 °C.

7. Appareil selon les revendications 1 à 5, **caractérisé par le fait que** l'élément chauffant (1) et le capteur de température (4) sont contenus dans un circuit intégré d'une puce de silicium (7) à transistor de commutation, l'élément chauffant (1) étant constitué d'une matrice de résistances (8).
